# EUROPEAN PATENT APPLICATION

(11) **EP 4 555 946 A1**
(43) Date of publication of application: **21.05.2025**
(21) Application number: 23209921.8
(22) Date of filing: 14.11.2023
(51) Int. Cl.: A61B 17/22, A61B 17/225

(54) **SUPPLEMENTARY ELEMENT FOR A SHOCKWAVE TREATMENT DEVICE, A SHOCKWAVE TREATMENT DEVICE AND A KIT INCLUDING SUCH A SUPPLEMENTARY ELEMENT**

(71) Applicant: Ferton Holding S.A., 2800 Delémont (CH)
(72) Inventor: FOURNIER, Maxime, 1010 Lausanne (CH); PIOZ, Hervé, 01630 Challex (FR)
(74) Representative: Müller Schupfner & Partner Patent- und Rechtsanwaltspartnerschaft mbB (Muc)

(57) **Abstract**

A supplementary element (1) configured to be attached to a shockwave treatment device (5), producing shockwave for treating a treatment zone of a patient in operation, the supplementary element being configured to allow for the transferring shockwave produced by the treatment device (5) to the treatment zone when the supplementary element (1) is attached to the treatment device (5), the supplementary element (1) further comprising a treatment support section (20), being configured to contact a periphery region of the patient surrounding at least partially the treatment zone, the treatment support section (20) being configured to allow for holding in position on the treatment zone the treatment device (5) in operation.

## Description

The present invention concerns a supplementary element for a shockwave treatment device, a shockwave treatment device and a kit including such a supplementary element.

Such shockwave treatment devices are intended to treat humans or animals by using shockwaves, the shockwaves being generated by an impact of an accelerated projectile on an impact body and are well known from the prior art. For example, EP 2 529 792 A1 and EP 2 381 864 B1 describe such shockwave treatment devices.

Typically, the shockwave treatment device has a pneumatic drive element for accelerating the projectile. In particular, the pneumatic drive element is intended to accelerate the projectile, which, in turn, hits the impact body, being in contact or close to the surface of the tissue, which should be treated. The shockwave, generated in the impact body, is transferred into the tissue of a patient by entering the body of the patient via the surface or skin, which is close to the impact body or is in contact with the impact body.

For supplying gas to a handpiece in order to accelerate the projectile, there is, in general, a compressor being part of the pneumatic drive element. Furthermore, the shockwave treatment device includes a control means for adjusting operating parameters of the pneumatic drive element, preferably a frequency and/or pressure. For example, EP 2 381 864 B1 suggests a compressor, which adapts its power to adjust the strength of the generated shockwave. Furthermore, it turned out that it is of advantage to use at least two compressors, which can be used to adjust the desired power level of the pressure drive device.

It was an objective technical problem to increase the comfort of both a patient, being treated with the shockwave treatment device, and the operator, using the shockwave treatment device, in particular, when it is used to treat an achille tendon.

This object is achieved by a supplementary element according to claim 1, a shockwave treatment device according to claim 14 and a kit according to claim 15. Preferred embodiments are incorporated in the dependent claims, the description and the figures.

According to a first aspect a supplementary element is provided the supplementary element being configured to be attached to a shockwave treatment device, producing shockwave for treating a treatment zone of a patient in operation, the supplementary element being configured to allow for transferring shockwave produced by the treatment device to the treatment zone when the supplementary element is attached to the treatment device, the supplementary element further comprising a treatment support section, being configured to contact a periphery region of the patient surrounding at least partially the treatment zone, the treatment support section being configured to allow for holding in position on the treatment zone the treatment device in operation.

In other words: A supplementary element for a shockwave treatment device is provided, preferably for a shockwave treatment device to treat an Achille tendon, the treatment device being configured to transfer a shockwave to a treatment zone of a patient in operation, wherein the supplementary element is connected to the shockwave treatment device in operation and comprises a treatment support section, being preferably configured to contact a periphery region of the treatment zone, the periphery region being located adjacent to the treatment zone in operation.

Contrary to the prior art, the present invention suggests a supplementary element having a treatment support section, being configured to contact a periphery region, the periphery region being located adjacent to the treatment zone in operation. As a result, it is possible that the operator attaches the distal end of the shockwave treatment device during operation in its position by using the treatment support sections, for example by pressing it against the periphery region. Therefore, a shift or a misalignment of the shockwave treatment device might be avoided, which otherwise could be issued due to a recoil experienced during the treatment. For example, the operator can determine the orientation, in particular of the distal end of the shockwave treatment device, by pressing the treatment support section to the periphery region, for example manually. Furthermore, it surprisingly turned out that a contact in the periphery region of the treatment zone reduces the pain of the patient, too. As a consequence, the comfort of both the patient and the operator during the use of the shockwave treatment device is improved.

In particular, in operation means that shockwaves are transferred to the treatment zone. Therefore, the phrase "in operation" preferable means the situation in which the shockwave treatment device is operated and localized such to the treatment zone that shockwaves are generated and transferred to the treatment zone of the patient, in particular such that the shockwave treatment device acts on the treatment zone.

Preferably, the shockwave treatment device is intended to be used for treating the Achille tendon. Due to the very specific geometry of this body part of a patient specific requirements need to be met for the treatment support section to contact the periphery region as being explained below. Preferably, it is provided that the periphery region is localized adjacent to the treatment zone in a direction extending horizontally. Preferably, it is provided that the treatment support section contacts periphery regions, in particular periphery regions being opposite to each other with respect to the treatment zone. In other words, the treatment zone is localized between two periphery areas or regions, which are contacted in operation by the treatment support sections.

Preferably, the treatment support section is configured such that it contacts the periphery region at least in an area extending 10 cm², preferably 15 cm², more preferably 20 cm² and most preferably 25 cm² . The treatment support section is preferably a flat body extending mainly along a plane.

The supplementary element can be integrated into the treatment device or can be attached to it. For example, it is conceivable that the shockwave treatment device is also intended to be used for other treatment zones besides the achille tendon and the operator just pulls over or attaches the supplementary element if they are interested in treating a patient with respect to its achille tendon.

In particular, it is provided that the treatment zone is an Achille tendon and the treatment support section is configured to be convertible between a non-operational configuration, in which the treatment support section is distant from the patient when the supplementary element is attached to the treatment device hold on the treatment zone, and an operational configuration, in which the treatment support section is being pressed against the periphery region while the treatment device is hold on the treatment zone.

Preferably, it is provided that the treatment support section is configured such that it can be transferred into an operational orientation/configuration, in which the treatment support section contacts the periphery region and in particular can be pressed against a periphery region in operation. In the following the terms configuration and orientations are used synonymously. Said very specific configuration of the treatment support section allows the operator to apply the supplementary element also in complex regions, such as in the region of the achille tendon, which might vary from patient to patient. To adapt these treatment support sections to the individual shape and geometry of the treatment zone and periphery zone of the patient, it is of advantage to have treatment support sections, which can be transferred into the operational orientation/configuration, in which they contact the periphery region and in particular can be pressed to it. In particular, in case of an achille tendon, the periphery regions and the treatment zones are mainly formed V-like, wherein the sides of the V-like geometry are differently angled for different patients. By using, for example, a hinge-like mechanism which allows pivoting the treatment support section, it is possible to transfer the support section into the correct position or orientation, which also allows pressing the treatment support section to the outside of the patient. Preferably, it is provided that at least two subsections of the treatment support section are orientated or configured such that it is possible to clamp the section of a patient between those subsections.

In particular, it is provided that the transfer between the non-operational configuration and the operational configuration is performed manually. In other words: it is provided that the transfer between the non-operational orientation and the operational orientation is performed manually. However, it is also conceivable that the transfer is at least supported by a further mechanical component and/or an actuator, which is used for performing the transfer to the correct position and/or for increasing the pressure to the periphery region via the treatment support sections.

Especially, it is provided that the treatment support section comprises at least one wing-like subsection being convertible from the non-operational configuration to the operational configuration when a pressure is applied on it by an operator. Preferably, it is provided that the treatment support section comprises at least one wing-like subsection, which can be transferred from a non-operational orientation/configuration to an operational orientation/configuration. Preferably, it is provided that by a thinning in the treatment support section, a hinge-like effect is introduced into the treatment support section and/or the wing-like subsection. Preferably, the treatment support section includes a bulge to simplify handling. Thus, it is possible to reorientate the wing-like subsection to the optimized orientation for contact and pressing the subsection to the periphery region.

Preferably, it is provided that the treatment support section has an arched front side in the non-operational configuration, the front side being directed towards the treatment zone in operationln particular, the front side faces the treatment zone and the periphery zone in operation. The arched course is preferably intended to support and simplify the movement from the non-operational configuration to the operational orientation/configuration, since it reduces the amount of newly orientating the wing-like subsections. Further the arched front side supports alignment of the shockwave treatment device having the supplementary element.

Preferably, it is provided that the supplementary element is made from an elastic material. This allows, for example, the transfer between the non-operational configuration and operational orientation and in particular the reuse of the supplementary element for another patient having another shape and geometry. Preferably, it is provided that the treatment support section is configured such that at least one subsection automatically returns into the non-operational configuration when the operator stops applying pressure on it.

In particular, it is intended that for example by a material distribution different elasticities are established in the supplementary element. For example, it is intended to create a thinned hinge-like component or subsection in the treatment support section for allowing the pivoting movement of the bulge-like wing subsection. On the other hand, the elasticity is reduced by thickening, for example, in the section, which intended for attaching the supplementary element to the shockwave treatment device to create a sufficiently strong connection, which is not affected by any recoil effect, back reflections and/or unintended contacts.

Preferably, it is provided that the supplementary element is a single-piece component. This is especially of advantage when the supplementary element is attached to the distal end of the shockwave treatment device. It supports a sufficient stability, being necessary since the supplementary element experiences counteracting forces during the operation. Furthermore, it guarantees the proper orientation of all components. A single-piece component cannot be divided without destroying or damaging the component.

Preferably, it is provided that the supplementary element has an interface section configured to cooperate with the treatment device to ensure the attachment of the supplementary element to the shockwave treatment device . In particular, it is provided that the distal end of the shockwave treatment device passes through the supplementary element in the mounted or attached status. In particular, the distal end of the shockwave treatment device is flush with the cause of the arched front side at the distal end of the supplementary element. Preferably, the interface section is formed as a tube, into which the shockwave treatment device can be inserted. In other words: the interface section has a tube-like form to pull it over the distal end of the shockwave treatment device. Furthermore, it is provided that the supplementary element is configured to be interchangeably attached to the shockwave treatment device. This allows the change of the supplementary element and furthermore also allows the use of the shockwave treatment device in different regions, while the supplementary element is preferably configured for a specific region, such as the achille tendon.

Preferably, it is provided that the treatment support section and/or the interface section has gripping means for allowing an operator of the treatment device to grip the supplementary element, for example formed by a recess. In particular, the tube-like interface section has at its outside at least one recess, for example a dimple-like or spherical-like recess to simplify the gripping. A groove, being for example formed at the front side of the treatment support section facing away from the treatment zone and the periphery zone, is intended for creating the hinge effect by thinning of the material as being mentioned.

Preferably, it is provided that the supplementary element has stabilization means for helping maintain the supplementary element on the treatment zone. For example, there is a protrusion which extends from the supplementary element to avoid in operation a pivoting or rotation of the shockwave treatment device along a plane extending vertically. Preferably, the treatment support section and the protrusion are orientated in an angled manner to each other, preferably by forming an angle being greater than 40°, more preferably more than 60° and most preferably more than 80°. Especially the treatment support section and the protrusion extend essentially perpendicular to each other.

Another aspect is a shockwave treatment device having a supplementary element according to the present invention attached to it. All specifications and benefits being discussed in context of the supplementary element applies analogously to the shockwave treatment device and vice versa. The supplementary element might be an integral part of the shockwave treatment device or is configured to be attached to the distal end of the shockwave treatment device.

Another aspect is a kit of supplementary elements for a shockwave treatment device, preferably with a colour code to specify the supplementary elements. All specifics and benefits being discussed previously in context of the supplementary element applies analogously to the kit and vice versa. In particular, it is intended to have different kinds of supplementary elements being preferably used in different treatment zones of the patient. By using, for example, a colour code, the operator can directly identify those supplementary elements, which are of benefit for the specified region, because of their specific shape of the treatment support section.

Wherever not already described explicitly, individual embodiments or their individual aspects and features can be combined or exchanged with one another without limiting or widening the scope of the described invention, whenever such a combination or exchange is meaningful and in the sense of this invention. Advantages which are described with respect to one embodiment of the present invention are, wherever applicable, also advantageous of other embodiments of the present invention.

In the drawings:
- **Fig. 1**: shows a supplementary element according to an exemplary embodiment of the present invention
- **Fig. 2**: shows a shockwave treatment device having a supplementary element (bottom) and having no supplementary element (top) and
- **Fig. 3**: shows further supplementary elements according to further embodiments of the present invention

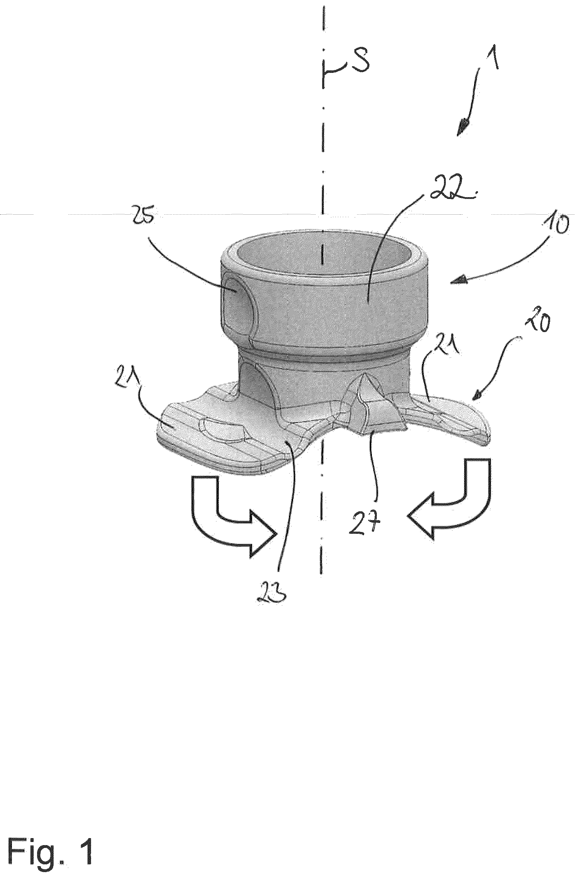

In **figure 1** a supplementary element 1 according to an exemplary embodiment of the present invention is illustrated. This supplementary element 1 does not contribute to the core process of a shock wave treatment device 5, but it turned out that the supplementary element 1, as shown in figure 1, simplifies the handling of the shockwave treatment device 5 and furthermore it surprisingly turned out that the use of this supplementary element 1 reduces a pain of a patient, being treated with the shockwave treatment device 5. This supplementary element 5 is, for example, integrated into the shockwave treatment device 5 or the shockwave treatment device 5 can be upgraded, for example, by attaching the supplementary element 1 to a distal end of the shockwave treatment device 5. The exemplary embodiment of figure 1 shows a supplementary element 1, which can be removably attached to the distal end of the shockwave treatment device 5.

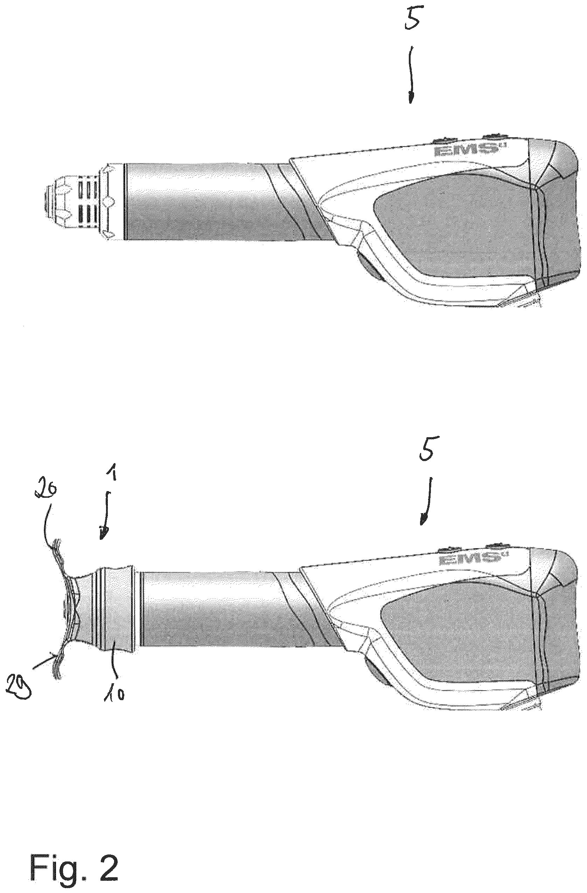

In particular, the supplementary element 1 comprises an interface section 10. As being shown in figure 1, this interface section 10 can be formed as a tube section, which can be pulled over a distal end of the treatment device 5 (a visualisation is shown in figure 2). In particular, the interface section 10 is configured such that the distal end of the shockwave treatment device 5 can reach through or pass through the hollow section of the tube forming the interface section 10. In the embodiment of figure 1 at the outside of the tube is at least 1 a recess 25 for simplifying and supporting gripping the supplementary element 1 manually. Preferably, there are at least two recesses 25 being located opposite to each other at the outside of the tube-like interface section 10. Furthermore, it is preferably provided that the recess 25 has a spherical-like or dimple-like geometry. This supports the comfort, when the operator grips the supplementary element 1 in this region.

Furthermore, the supplementary element 1 comprises a treatment support section 20. The treatment support section 20 of the supplementary element 1 is directly attached to the tube-like interface section 10. In particular, the supplementary element 1 of figure 1 has an interface section 10 including a collar-like section 22 of a thickness being larger than the thickness of the region of the tube, which connects the interface section 10 and the treatment support section 20 .

The treatment support section 20 is configured such that it contacts a periphery region being located next to a treatment zone, which is addressed by the shockwave's treatment device 5. The treatment zone is defined by the region of the patient, which is affected by an impact body that hits the treatment zone. By using a treatment support section 20, which contacts the periphery region of the treatment zone, it is advantageously possible to stabilize the shockwave treatment device 5, which itself experiences a recoil during the treatment. By pressing the treatment support section 20 to the periphery region, preferably manually, it is possible to avoid any shift of the shockwave treatment device 5, which otherwise occur, when the shockwave treatment device 5 is used.

In particular, the supplementary element 1 is intended to be used for a shockwave treatment device 5 when it is applied to a treatment zone, being localized in the region of the achilles heifer.

Due to the specific geometry of the achilles heifer, the supplementary element 1 being illustrated in figure 1 is configured such that the treatment support section 20 can be transferred between an operational configuration and a non-operational configuration. This allows to transfer the treatment support sections 20 such that it can contact the periphery region of the patient and in particular can be pressed against the periphery region, in particular independent of the size and geometry of the achilles heifer of the patient. In particular, the skilled person knows that the achilles heifer is treated by the shockwave treatment device 5 such that the shockwave treatment device 5 is localized at the rear end of the foot, in particular in the region of the achilles heifer. Especially, a cross-section, which includes the periphery region, adjacent to the treatment zone, and the rear section of the foot, i. e. the treatment zone, is mainly V-shaped such that the transfer allows to contact the tip of the V-like section with the distal end of the shockwave treatment device 1, while the treatment support sections 20 can contact the sides of the V-like geometry. Thus, it is possible to contact a comparably large area by the treatment support section 20, in particular in an area being significantly larger than the contact region between the treatment zone and the distal end of the shockwave treatment device 5. In particular, the treatment support section 20 is configured such that in operation the treatment support section 20 essentially extends horizontally. Furthermore, a symmetry axis S can be assigned to the interface section 10.

In the embodiment of figure 1, the treatment support section 20 has at least one wing-like subsection, preferably two wing-like subsections, which are localized at opposite sides with respect to the symmetry axis S. The symmetry axis S is preferably defined by a direction, along which the shockwave treatment device 5 is inserted into the supplementary element 1. In particular, it is provided that the wing-like subsection can be pivoted along an axis being mainly perpendicular to the symmetry axis S of the supplementary element 1. Preferably, it is provided that for creating a hinge-like effect, the wing-like subsection includes a groove 23, which is formed, for example by a thinning of the treatment support section 20. Thus, it is possible to use this reduction in thickness for a hinge effect, which allows the transfer between the non-operational configuration and the operational orientation. At the end of the wing-like subsections, a bulge 21 is formed to simplify the handling, when an operator presses the wing-like subsections to the periphery region of the patient.

In particular, the bulge 21 controls the direction of the forces, when the treatment support section 20 is pressed against the periphery region.

Furthermore, the supplementary element 1 includes a stabilization element, being formed as a protrusion 27, which extends perpendicular to the symmetry axis S. In particular, the preferably nose-like protrusion 27 is formed at the front side 29 of the supplementary element, being directed to the treatment zone and the periphery zone during the operation. The stabilization element extends from the symmetry axis S in a direction, being perpendicular to the symmetry axis S and is preferably rotated about 90° to the direction being defined by the extension of the wing-like subsections Thus, it is possible that this stabilizing element extends mainly vertically in operation and thus also stabilizes the shockwave treatment device 5 in an additional direction, since it hinders the shockwave treatment device 5 to perform a movement along a vertically extending plane. A radial extension of the protrusion 27 is smaller, preferably two times smaller, more preferably five times smaller and most preferably more than eight times smaller than the radial extension of the wing-like subsection. A measurement of the extension starts at the (thought) outer side of the tube forming part of the interface section 10.

In contrast to the protrusion 27, the treatment support section 20 extends during operation mainly horizontally, such that the wing-like subsections of the treatment support section 20 counteract a shift or a misorientation in a horizontal plane in operation.

Furthermore, it is intended that the supplementary element 1 is formed as a single piece element, being made preferably by plastic, in particular a plastic material, in particular a rubber material being elastic.

In **figure 2** a shockwave treatment device 5 according to an exemplary embodiment is shown. Such a shockwave treatment device 5 is intended to treat tissue of a human body or an animal by generating a shockwave, which acts on the tissue of the body. In particular, it is provided that the shockwave treatment device 5 is located on the skin of the human or animal body and the shockwave enters the human or animal body through the skin and acts on tissue inside of the human or animal body. Thereby, the shockwave treatment device 5 includes an impact body, which is in contact with the skin of the human or animal during the treatment. For generating the shockwave, the shockwave treatment device 5 includes a projectile, which is accelerated in a pipe for guiding the projectile. The accelerated projectile hits on the impact body, generating the shockwave being emitted by the shockwave treatment device 5. In particular, the shockwave treatment device 5 has a handpiece, including at least a pipe for accelerating the projectile, and a pneumatic drive element, which provides pressurized gas to accelerate the projectile inside the pipe, in which the projectile is accelerated. Furthermore, it is conceivable that the shockwave treatment device 5 has a regulation unit. By actuating the regulation unit, it is possible to define parameters of operation of the shockwave treatment device.

In the illustration at the bottom side, the shockwave treatment device is shown with an attached supplementary element 1. In this illustration, it is shown that the front side 29 which faces the treatment zone during operation, has an arched shape in the non-operational configuration or status. This simplifies the start positioning compared to the arched curves in the non-operational configuration as being shown in the bottom figure of figure 2 and in the operational configuration, the wing-like sections are pivoted such that the front side 29 is arched to a greater extent or amount.

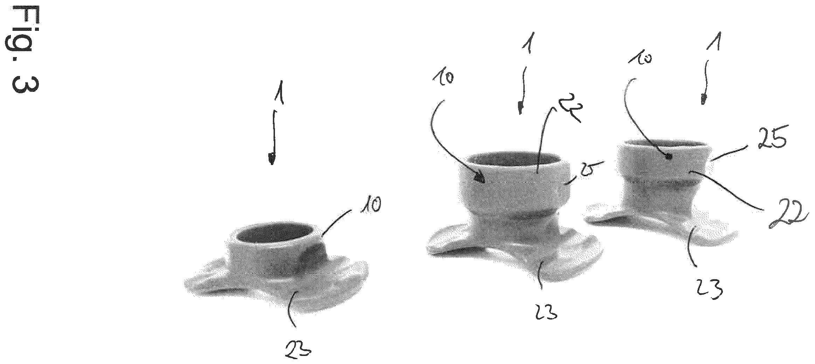

In **figure 3** further exemplary embodiments of a supplementary element 1 according to an exemplary embodiment of the present invention are illustrated. It is shown that the height of the interface section 10, in particular of the collar-like section 22, at the topside of the supplementary element can have different sizes. The thickness variation in the interface section 10 can even avoid in some embodiments, as being illustrated on the left side embodiment.

### Reference signs:

- 1: Supplementary element
- 5: Shockwave treatment device
- 10: Interface section
- 20: Treatment support section
- 21: Bulge
- 22: Collar-like section
- 23: Groove
- 25: Recess
- 27: Protrusion
- 29: Front side
- S: Symmetry axis

## Claims

1. A supplementary element (1) configured to be attached to a shockwave treatment device (5), producing shockwave for treating a treatment zone of a patient in operation, the supplementary element being configured to allow for transferring shockwave produced by the treatment device (5) to the treatment zone when the supplementary element (1) is attached to the treatment device (5), the supplementary element (1) further comprising a treatment support section (20), being configured to contact a periphery region of the patient surrounding at least partially the treatment zone, the treatment support section (20) being configured to allow for holding in position on the treatment zone the treatment device (5) in operation.

2. The supplementary element (1) of claim 1, wherein the treatment zone is an Achille tendon and wherein the treatment support section (20) is configured to be convertible between a non-operational configuration, in which it is distant from the patient when the supplementary element is attached to the treatment device hold on the treatment zone, and an operational configuration, in which the treatment support section (20) is being pressed against the periphery region while the treatment device (5) is hold on the treatment zone.

3. The supplementary element (1) according to claim 2, wherein the treatment support section (20) comprises at least one wing-like subsection being convertible from the non-operational configuration to the operational configuration when a pressure is applied on it by an operator.

4. The supplementary element (1) according to claim 3, wherein the treatment support section (20) is configured such that the at least one wing-like subsection automatically returns into the non-operational configuration when the operator stops applying pressure on it.

5. The supplementary element (1) according to one of the preceding claims,
wherein the treatment support section (20) has an arched front side (29) in the non-operational configuration, the front side (29) being directed towards the treatment zone in operation.

6. The supplementary element (1) according to one of the preceding claims,
wherein the supplementary element (1) is made from an elastic material.

7. The supplementary element (1) according to one of the preceding claims,
wherein the supplementary element (1) is a single-piece component.

8. The supplementary element (1) according to one of the preceding claims,
wherein the supplementary element (1) has an interface section (10), configured to cooperate with the treatment device (5) to ensure the attachment of the supplementary element (1) to the shockwave treatment device (5).

9. The supplementary element (1) according to one of the preceding claims,
wherein the interface section (10) is configured to attach the supplementary element (1) to the shockwave treatment device (5) by force fitting means, form fitting means and/or adhesive fitting means.

10. The supplementary element (1) according one of the preceding claims, wherein the supplementary element (1) is configured to be interchangeably attached to the shockwave treatment device (5).

11. The supplementary element (1) according to claim 9 or 10, wherein the interface section (10) has a tube-like form to pull it over the distal end of the shockwave treatment device (5).

12. The supplementary element (1) according to one of the preceding claims,
wherein the treatment support section (20) and/or the interface section (10) has gripping means for allowing an operator of the treatment device (5) to grip the supplementary element (1), for example formed by a recess (25).

13. The supplementary element (1) according to one of the preceding claims,
wherein the supplementary element (1) has a stabilization means for helping maintain the supplementary element (1) on the treatment zone, being preferably formed as protrusion (27).

14. A shockwave treatment device (5) having a supplementary element (1) according to one of the preceding claims attached to it.

15. A kit of supplementary elements (1) for a shockwave treatment device (5), preferably with a color code to specify the supplementary elements (1).
